# EUROPEAN PATENT APPLICATION

(11) **EP 4 183 803 A1**
(43) Date of publication of application: **24.05.2023**
(21) Application number: 21841750.9
(22) Date of filing: 14.07.2021
(51) Int. Cl.: C07K 16/28, C12N 15/62, A61K 38/00, A61P 37/08, A61K 39/00

(54) **FUSION PROTEIN COMPRISING IGE FC RECEPTOR ALPHA SUBUNIT EXTRACELLULAR DOMAIN AND ANTI-IL-4R ANTIBODY, AND USE THEREOF**

(30) Priority: 17.07.2020 KR 20200088941
(71) Applicant: GI Innovation, Inc., Seoul 05855 (KR)
(72) Inventor: JANG, Myung Ho, Seoul 05849 (KR); NAM, Su Youn, Seoul 05855 (KR); CHO, Young-Gyu, Daejeon 34032 (KR); OH, Young Min, Yongin-si Gyeonggi-do 16847 (KR); LEE, Kyungwha, Uiwang-si Gyeonggi-do 16034 (KR); OH, Nahyun, Seongnam-si Gyeonggi-do 13477 (KR)
(74) Representative: HGF
(86) International application number: PCT/KR2021/009047
(87) International publication number: WO 2022/015049

(57) **Abstract**

The present invention relates to a fusion protein dimer comprising an IgE Fc receptor alpha subunit extracellular domain (FcεRIα-ECD) and a fragment of anti-IL-4R antibody; and a composition for treating allergic diseases, comprising the same. The fusion protein dimer according to the present invention exhibits an excellent IgE-binding ability and an excellent serum IgE level-reducing effect. In addition, the present invention elicits a hyperimmune response of IgE, and thus has an excellent effect of inhibiting the activity of cytokines inducing allergic diseases, such as IL-4 and IL-13, and thus may be applied as a medicine for the use of treating or preventing IgE-mediated allergic diseases.

## Description

### Technical Field

The present invention relates to a fusion protein dimer comprising an IgE Fc receptor alpha subunit extracellular domain (FcεRIα-ECD) and a fragment of anti-IL-4R antibody; and a composition for treating allergic diseases, comprising the same.

### Background Art

Allergic diseases, such as allergic rhinitis, atopic dermatitis, and food allergy, including asthma are increasing rapidly in industrialized and westernized modern societies, and the occurrence of anaphylaxis, a severe allergic disease, is also increasing. These chronic immune diseases severely impair individual's quality of life and socioeconomic costs are soaring accordingly. Thus, there is a desperate need for measures to overcome such diseases.

Most allergic diseases are caused by an excessive immune response of immunoglobulin E (IgE). IgE is an antibody that is present in serum at a very low concentration under a normal condition. IgE is also usually produced by innocuous antigens. There is a case where the number of IgE is increased without any particular stimulus. Such a case may lead to allergic diseases. The abnormally increased number of IgE can bind to the high affinity IgE Fc receptor (FcεRI) which are expressed on the surface of mast cells, basophils and the like. Such binding causes mast cells or basophils to release chemical mediators such as histamine, leukotriene, prostaglandin, bradykinin and platelet-activating factor. Release of these chemical mediators results in allergic symptoms. In particular, allergic diseases may exhibit worsened symptoms due to the binding between IgE and FcεRI.

Currently, various methods, such as allergen avoidance, administration of anti-allergic drugs, modulation of IgE synthesis in the body, and development of anti-IgE antibodies, have been proposed to treat allergic diseases. However, therapeutic methods known so far have many drawbacks, such as inability to cure an underlying cause of allergy, insufficient drug efficacy, and occurrence of serious side effects.

On the other hand, the biological activity of the inflammatory cytokine IL-4 is mediated by a specific IL-4 receptor (interleukin-4 receptor, IL-4R) on the cell surface. There are two types of IL-4 receptors: type 1 in which the IL-4 receptor α chain and γc chain are complexed, and type 2 in which the IL-4 receptor α chain and the IL-13 receptor α1 chain are complexed. In this regard, it has been demonstrated that human monoclonal antibodies against the IL-4Rα chain are clinically effective in alleviating and treating symptoms such as asthma, eczema, and atopic dermatitis.

Until now, the anti-hIL-4Rα antibody, dupilumab, developed by Regeneron Pharmaceuticals Inc., was approved by the FDA in 2017, and is being used to treat allergic diseases (US Patent No. 7,605,237). The anti-hIL-4Rα antibodies other than dupilumab have not yet been approved.

### Detailed Description of Invention

### Technical Problem

Accordingly, the present inventors studied to develop a new combination of fusion proteins for effectively treating and preventing allergic diseases. As a result, it was confirmed that a fusion protein dimer comprising an IgE Fc receptor alpha subunit extracellular domain (FcεRIα-ECD) and a fragment of anti-IL-4R antibody exhibits an excellent IgE-binding ability and an excellent serum IgE level-reducing effect. In addition, it was confirmed that the activity of IL-4 and IL-13, cytokines that elicit a hyperimmune response of IgE to induce allergic diseases, is inhibited in a concentration-dependent manner. Based on the above, the present invention was completed by identifying that the fusion protein dimer can be usefully utilized as a therapeutic agent for allergic diseases such as asthma and atopic dermatitis.

### Solution to Problem

In order to achieve the above object, in an aspect of the present invention, there is provided a fusion protein dimer comprising an IgE Fc receptor alpha subunit extracellular domain (FcεRIα-ECD) and a fragment of anti-IL-4R antibody.

In another aspect of the present invention, there is provided a polynucleotide encoding the fusion protein, an expression vector comprising the polynucleotide, and a transformed cell into which the expression vector is introduced.

In another aspect of the present invention, there is provided a method of producing a fusion protein dimer, comprising: culturing the transformed cell; and recovering a fusion protein dimer.

In another aspect of the present invention, there is provided a pharmaceutical composition for preventing or treating allergic diseases, comprising the fusion protein dimer.

In another aspect of the present invention, there is provided a food composition for improving or alleviating allergic symptoms, comprising the fusion protein dimer.

In another aspect of the present invention, there is provided a use of a fusion protein dimer comprising an FcεRIα-ECD and a fragment of anti-IL-4R antibody for the manufacture of a medicament for the treatment or prevention of allergic diseases.

In another aspect of the present invention, there is provided a use of a fusion protein dimer comprising an FcεRIα-ECD and a fragment of anti-IL-4R antibody for the treatment or prevention of allergic diseases.

In another aspect of the present invention, there is provided a method for treating or preventing allergic diseases, comprising administering to a subject the fusion protein dimer comprising an FcεRIα-ECD and a fragment of anti-IL-4R antibody.

### Effects of Invention

The fusion protein dimer according to the present invention comprising an IgE Fc receptor alpha subunit extracellular domain (FcεRIα-ECD) and a fragment of anti-IL-4R antibody exhibits an excellent IgE-binding ability and an excellent serum IgE level-reducing effect. In addition, it elicits a hyperimmune response of IgE, and thus has an excellent effect of inhibiting the activity of cytokines inducing allergic diseases, such as IL-4 and IL-13. In addition, in a functional aspect, the fusion protein dimer acts complexly as an IgE Trap and an anti-IL-4R antibody, and thus it can be usefully utilized as a new pharmaceutical composition for the treatment of allergic diseases that can replace individual therapeutic agents of conventional anti-IgE antibodies and anti-IL-4R antibodies.

### Brief Description of Drawings

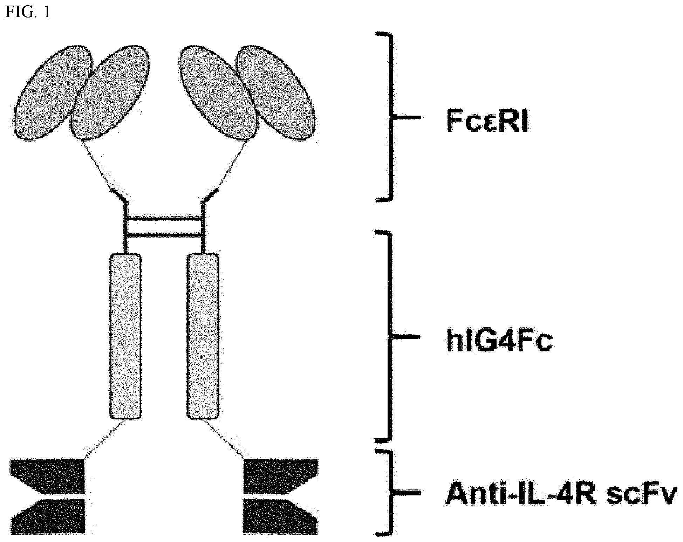

FIG. 1 illustrates the structure of the fusion protein GI-305 according to the present invention (FcεRIα ECD-Fc-anti-IL-4R scFv).
FIG. 2 illustrates the results obtained by identifying the obtained fusion protein GI-305 by SDS-PAGE.
FIG. 3 illustrates the results obtained by identifying the obtained fusion protein GI-305 by Western blot.
FIG. 4 is a graph showing the results obtained by measuring the binding ability of the fusion protein GI-305 according to the present invention to human IgE.
FIG. 5 is a graph showing the results obtained by identifying the efficacy of the fusion protein GI-305 according to the present invention as an anti-IL-4R antibody.
FIGs. 6 and 7 are graph showing the results obtained by identifying the serum IgE level-reducing effect of the fusion protein GI-305 according to the present invention.

### Best Mode for Carrying out the Invention

### Fusion protein comprising FcεRIα-ECD and fragment of anti-IL-4R antibody

In an aspect of the present invention, there is provided a fusion protein comprising an IgE Fc receptor alpha subunit extracellular domain (FcεRIα-ECD) and a fragment of anti-IL-4R antibody.

As used herein, the term "IgE" means an antibody protein known as immunoglobulin E. IgE has affinity to mast cells, basophils in blood, or the like. In addition, reaction between an IgE antibody and an antigen (allergen) corresponding thereto causes an inflammatory reaction. In addition, IgE is known to be an antibody that causes anaphylaxis which occurs due to sudden secretion of mast cells or basophils.

As used herein, the term "IgE Fc receptor" is also referred to as Fcε receptor, and binds to an Fc portion of IgE. There are two types for the receptors. The receptor having high affinity to IgE Fc is called Fcε receptor I (FcεRI). The receptor having low affinity to IgE Fc is called Fcε receptor II (FcεRII). FcεRI is expressed in mast cells and basophils. In a case where IgE antibodies bound to FcεRI are cross-linked by polyvalent antigens, degranulation occurs in mast cells or basophils, thereby releasing various chemical transmitter substances including histamine. This release leads to an immediate allergic reaction.

The FcεRI is a membrane protein composed of one α chain, one β chain, and two γ chains linked by a disulfide bond. Among these chains, a portion to which IgE binds is the α chain (FcεRIa), and FcεRIα has a size of about 60 kDa, and is composed of a hydrophobic domain existing inside the cell membrane and a hydrophilic domain existing outside the cell membrane. In particular, IgE binds to the extracellular domain of the α chain. The "FcεRIα" may be used interchangeably with "IgE Fc receptor alpha subunit."

Specifically, the alpha subunit of the IgE Fc receptor may have the amino acid sequence set forth in NP_001992.1. In addition, the extracellular domain of the alpha subunit of the IgE Fc receptor (FcεRIα-ECD) may have the amino acid sequence of SEQ ID NO: 2. In the present specification, the extracellular domain of the alpha subunit of the IgE Fc receptor may be a fragment or variant of the extracellular domain of the alpha subunit of the IgE Fc receptor, as long as the fragment or variant is capable of binding to IgE.

The variant may be prepared through a method of substituting, deleting, or adding one or more proteins in the wild-type FcεRIα-ECD (extracellular domain), as long as the method does not alter a function of the α chain of FcεRI. Such various proteins or peptides may be 90%, 91%, 92%, 93%, 94%, 95%, 96%, 97%, 98%, 99% or more identical to the amino acid sequence of SEQ ID NO: 2. In addition, FcεRIα ECD of SEQ ID NO: 2 may be encoded by a polynucleotide having the sequence of SEQ ID NO: 11.

As used herein, the term "IL-4R (interleukin-4 receptor)" or "interleukin-4 receptor" is a cytokine receptor to which interleukin-4 (IL-4) specifically binds, and exists as a type 1 IL-4 receptor and a type 2 IL-4 receptor. The type 1 IL-4 receptor is a dimer receptor comprising an IL-4Rα chain and a γc chain, and the type 2 IL-4 receptor is a dimer receptor comprising an IL-4Rα chain and an IL-13Rα1 chain. The type 1 IL-4 receptor interacts with and is stimulated by IL-4, and the type 2 IL-4 receptor interacts with and is stimulated by both IL-4 and IL-13.

The Th2 cytokines, IL-4 and IL-13, are known to play an important role in allergic diseases such as asthma, atopic dermatitis, and allergic rhinitis, and are involved in the growth of T lymphocytes, differentiation into Th2 cells, and conversion of B lymphocyte products into IgE. In addition, IL-4 and IL-13 share IL-4 receptor α (IL-4 Rα, IL-4 receptor α) and have similar biological properties. Specifically, IL-4 is essential for the conversion of B lymphocyte products into IgE, and promotes chemotaxis and adhesion of eosinophil. In addition, IL-13 acts as an important mediator for IgE production and maintenance. Therefore, IL-4 and IL-13 elicit a hyperimmune response of IgE to induce allergic diseases.

Here, the anti-IL-4R antibody may be an antibody that specifically binds to the IL-4 receptor. Specifically, it may be an antibody that specifically binds to the type 2 IL-4 receptor. In addition, the fragment of antibody may be used in any form as long as it comprises an antigen binding domain capable of specifically binding to an IL-4 receptor. In addition, the fragment of anti-IL-4R antibody may be in the form of an scFv. As used herein, the term "scFv" is an abbreviation of a single chain variable fragment, and refers to a form in which a heavy chain variable region and a light chain variable region are combined by a peptide linker.

The heavy chain variable region of the scFv of the anti-IL-4R antibody used in an embodiment of the present invention may have the amino acid sequence of SEQ ID NO: 7 or 26. Specifically, HCDR1, HCDR2 and HCDR3 of the heavy chain variable region may be SEQ ID NOs: 20, 21 and 22, respectively. In addition, the light chain variable region of the scFv of the anti-IL-4R antibody may have the amino acid sequence of SEQ ID NO: 8 or 27. Specifically, LCDR1, LCDR2 and LCDR3 of the light chain variable region may be SEQ ID NOs: 23, 24 and 25, respectively. In addition, in an embodiment, the peptide linker that links the heavy chain variable region and the light chain variable region may have the amino acid sequence of SEQ ID NO: 9. In addition, the scFv of the anti-IL-4R antibody used in an embodiment of the present invention may have the amino acid sequence of SEQ ID NO: 6.

On the other hand, the heavy chain variable region of the scFv of the anti-IL-4R antibody of SEQ ID NO: 7 used in an embodiment of the present invention may be one obtained by mutating the 44th amino acid from G into C in the amino acid sequence of dupilumab scFv (SEQ ID NO: 26) for the stabilization of the scFv structure through disulfide bond formation. In addition, the light chain variable region of the scFv of the anti-IL-4R antibody of SEQ ID NO: 8 used in an embodiment of the present invention may be one obtained by mutating the 105th amino acid from Q into C in the amino acid sequence of dupilumab scFv (SEQ ID NO: 27) for the stabilization of the scFv structure through disulfide bond formation.

Here, the fusion protein may contain an Fc region of an immunoglobulin. Here, the Fc domain of an immunoglobulin refers to a protein that contains a heavy chain constant region 2 (CH2) and a heavy chain constant region 3 (CH3) of an immunoglobulin, but does not contain heavy and light chain variable regions and a light chain constant region (CL) of an immunoglobulin. The immunoglobulin may be IgG, IgA, IgE, IgD or IgM, and may be preferably IgG4.

In addition, the Fc domain of an immunoglobulin may be a wild type Fc domain as well as an Fc domain variant. In addition, as used herein, the term "Fc domain variant" may refer to a form which is different from the wild type Fc domain in terms of glycosylation pattern, or has a high glycosylation as compared with the wild type Fc domain, or has a low glycosylation as compared with the wild type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD or IgM. In addition, the Fc domain variant may be in a form in which some amino acids of the Fc domain are substituted with other amino acids.

In addition, the modified Fc region may have glycosylation of the native form or have a high glycosylation as compared with the native form. Glycosylation of the Fc domain of an immunoglobulin may be modified by conventional methods such as chemical methods, enzymatic methods, and genetic engineering methods using microorganisms.

In an embodiment, the Fc domain variant may have the amino acid sequence of SEQ ID NO: 4.

Specifically, the fusion protein may consist of the following structural formula (I) or (II):

N'-X-linker (1)-Fc region fragment or variant thereof-linker (2)-Y-C' (I)

N'-Y-linker (1)-Fc region fragment or variant thereof-linker (2)-X-C' (II)

in the structural formulas (I) and (II),
N' is the N-terminus of the fusion protein,
C' is the C-terminus of the fusion protein,
X is FcεRIα-ECD or a fragment thereof,
Y is a fragment of anti-IL-4R antibody, and
the linker (1) and linker (2) are peptide linkers.

Here, the FcεRIα-ECD or fragment thereof and the fragment of anti-IL-4R antibody are as described above.

Here, the peptide linker (1) may consist of 5 to 80 consecutive amino acids, 10 to 70 consecutive amino acids, 15 to 60 consecutive amino acids, 20 to 50 consecutive amino acids, 25 to 40 consecutive amino acids, or 25 to 35 amino acids. In an embodiment, the peptide linker (1) may consist of 30 amino acids. In addition, the peptide linker (1) may comprise at least one cysteine. Specifically, it may comprise one, two or three cysteines. In addition, the peptide linker (1) may be derived from the hinge of an immunoglobulin. In an embodiment, the peptide linker (1) may be a peptide linker consisting of the amino acid sequence of SEQ ID NO: 3.

The peptide linker (2) may consist of 1 to 50 consecutive amino acids, or 3 to 30 consecutive amino acids, or 5 to 15 amino acids. The peptide linker (2) may include (G₄S)n (wherein, n is an integer from 1 to 10), and may further include (G)n. Here, in (G₄S)n and (G)n, n may be 1, 2, 3, 4, 5, 6, 7, 8, 9 or 10, respectively. The peptide linker (2) may be a peptide linker consisting of the amino acid sequence of SEQ ID NO: 5.

Each amino acid sequence of the fusion protein is as shown in Table 1 below. In addition, in an embodiment, the amino acid sequence of the GI-305 (FcεRIα ECD-Fc-anti-IL-4R scFv) may have the amino acid sequence of SEQ ID NO: 10.

**[Table 1]**

| Item | Amino acid sequence | SEQ ID NO |
|---|---|---|
| FcεRIα ECD | VPQKPKVSLNPPWNRIFKGENVTLTCNGN NFFEVSSTKWFHNGSLSEETNSSLNIVNAK FEDSGEYKCQHQQVNESEPVYLEVFSDW LLLQASAEVVMEGQPLFLRCHGWRNWD VYKVIYYKDGEALKYWYENHNISITNATV EDSGTYYCTGKVWQLDYESEPLNITVIKA PREKYWLQ | 2 |
| First linker | GSGGGGSGGGGSGGGGSAESKYGPPCPPC P | 3 |
| hIgG4Fc | APEAAGGPSVFLFPPKPKDQLMISRTPEVT CVVVDVSQEDPEVQFNWYVDGVEVHNA KTKPREEQFNSTYRVVSVLTVLHQDWLN GKEYKCKVSNKGLPSSIEKTISKAKGQPRE PQVYTLPPSQEEMTKNQVSLTCLVKGFYP SDIAVEWESNGQPENNYKTTPPVLDSDGS FFLYSRLTVDKSRWQEGNVFSCSVLHEAL HNHYTQKSLSLSLG | 4 |
| Second linker | GGGGSGGGGSGG | 5 |
| Anti-IL-4R scFv | EVQLVESGGGLEQPGGSLRLSCAGSGFTF RDYAMTWVRQAPGKCLEWVSSISGSGGN TYYADSVKGRFTISRDNSKNTLYLQMNSL RAEDTAVYYCAKDRLSITIRPRYYGLDVW GQGTTVTVSSGGGGSGGGGSGGGGSGGG GSDIVMTQSPLSLPVTPGEPASISCRSSQSL LYSIGYNYLDWYLQKSGQSPQLLIYLGSN RASGVPDRFSGSGSGTDFTLKISRVEAEDV GFYYCMQALQTPYTFGCGTKLEIK | 6 |
| Anti-IL-4R_heavy chain | EVQLVESGGGLEQPGGSLRLSCAGSGFTF RDYAMTWVRQAPGKCLEWVSSISGSGG NTYYADSVKGRFTISRDNSKNTLYLQMNS LRAEDTAVYYCAKDRLSITIRPRYYGLD VWGQGTTVTVSS | 7 |
| Anti-IL-4R_light chain | DIVMTQSPLSLPVTPGEPASISCRSSQSLLY SIGYNYLDWYLQKSGQSPQLLIYLGSNRA SGVPDRFSGSGSGTDFTLKISRVEAEDVGF YYCMQALQTPYTFGCGTKLEIK | 8 |
| HC-LC linker | GGGGSGGGGSGGGGSGGGGS | 9 |
| GI-305 (FcεRIα ECD-Fc-anti-IL-4R scFv) | VPQKPKVSLNPPWNRIFKGENVTLTCNGN NFFEVSSTKWFHNGSLSEETNSSLNIVNAK FEDSGEYKCQHQQVNESEPVYLEVFSDW LLLQASAEVVMEGQPLFLRCHGWRNWD VYKVIYYKDGEALKYWYENHNISITNATV EDSGTYYCTGKVWQLDYESEPLNITVIKA PREKYWLQ GSGGGGSGGGGSGGGGSAESKYGPPCPPC P APEAAGGPSVFLFPPKPKDQLMISRTPEVT CVVVDVSQEDPEVQFNWYVDGVEVHNA KTKPREEQFNSTYRVVSVLTVLHQDWLN GKEYKCKVSNKGLPSSIEKTISKAKGQPRE PQVYTLPPSQEEMTKNQVSLTCLVKGFYP SDIAVEWESNGQPENNYKTTPPVLDSDGS FFLYSRLTVDKSRWQEGNVFSCSVLHEAL HNHYTQKSLSLSLG | 10 |
| | GGGGSGGGGSGG EVQLVESGGGLEQPGGSLRLSCAGSGFTF RDYAMTWVRQAPGKCLEWVSSISGSGGN TYYADSVKGRFTISRDNSKNTLYLQMNSL RAEDTAVYYCAKDRLSITIRPRYYGLDVW GQGTTVTVSS GGGGSGGGGSGGGGSGGGGS DIVMTQSPLSLPVTPGEPASISCRSSQSLLY SIGYNYLDWYLQKSGQSPQLLIYLGSNRA SGVPDRFSGSGSGTDFTLKISRVEAEDVGF YYCMQALQTPYTFGCGTKLEIK | |

### Fusion protein dimer

In another aspect of the present invention, there is provided a fusion protein dimer in which two fusion proteins as described above are combined. Here, the fusion protein may be combined by a cysteine contained in a linker that links an FcεRIα-ECD or a fragment thereof with an Fc region of an immunoglobulin. Here, the linker may comprise a hinge region of an immunoglobulin.

### Polynucleotide encoding fusion protein

In another aspect of the present invention, there is provided a polynucleotide encoding the fusion protein comprising an FcεRIα-ECD and a fragment of anti-IL-4R antibody. The FcεRIα-ECD and fragment of anti-IL-4R antibody are as described above.

The polynucleotide may have the sequence of SEQ ID NO: 19.

If the polynucleotide encodes the same polypeptide, one or more nucleotides may be mutated by substitution, deletion, insertion, or a combination thereof. When the polynucleotide sequence is prepared by chemical synthesis, a synthesis method well known in the art, for example, the method described in the literature (Engels and Uhlmann, Angew Chem IntEd Engl., 37:73-127, 1988), may be used, and may include triester, phosphite, phosphoramidite and H-phosphate methods, PCR, and other autoprimer methods, oligonucleotide synthesis methods on a solid support, and the like.

According to an embodiment, the polypeptide may include a nucleic acid sequence having at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% identity to the nucleotide sequence of SEQ ID NO: 19.

The polynucleotide may further comprise a signal sequence or a leader sequence. As used herein, the term "signal sequence" refers to a nucleic acid encoding a signal peptide that directs secretion of a target protein. The signal peptide is translated and then cleaved in a host cell. Specifically, the signal sequence of the present invention is a nucleotide encoding an amino acid sequence that initiates migration of a protein across the endoplasmic reticulum (ER) membrane.

The signal sequences are well known in the art for their characteristics. Such signal sequences typically contain 16 to 30 amino acid residues, and may contain more or fewer amino acid residues than such amino acid residues. A typical signal peptide consists of three regions which are a basic N-terminal region, a central hydrophobic region, and a more polar C-terminal region. The central hydrophobic region contains 4 to 12 hydrophobic residues that immobilize the signal sequence through the membrane lipid bilayer during translocation of an immature polypeptide.

After initiation, signal sequences are cleaved in the lumen of ER by cellular enzymes, commonly known as signal peptidases. Here, the signal sequence may be a secretory signal sequence of tissue plasminogen activator (tPa), Herpes simplex virus glycoprotein D (HSV gD), IgG signal sequence, or a growth hormone. Preferably, a secretory signal sequence used in higher eukaryotic cells including mammals and the like, can be used.

Useful signal sequences in the present invention include antibody light chain signal sequences, for example, antibody 14.18 (Gillies et al., J. Immunol. Meth 1989. 125:191-202), antibody heavy chain signal sequences, for example, the MOPC141 antibody heavy chain signal sequence (Sakano et al., Nature, 1980. 286: 676-683), and other signal sequences known in the art (see, for example, Watson et al., Nucleic Acid Research, 1984. 12:5145-5164). In an embodiment, a signal sequence composed of amino acids of SEQ ID NO: 1 may be used as a signal sequence.

### Vector loaded with polynucleotide

In another aspect of the present invention, there is provided an expression vector comprising a polynucleotide encoding a fusion protein comprising an FcεRIα-ECD and a fragment of anti-IL-4R antibody. Here, the polynucleotide may have the sequence of SEQ ID NO: 19.

As used herein, the term "vector" is intended to be introduced into a host cell and to be capable of being recombined with a genome of the host cell and inserted thereinto. Alternatively, the vector is understood as nucleic acid means containing a nucleotide sequence which is autonomously replicable as an episome. The vectors include linear nucleic acids, plasmids, phagemids, cosmids, RNA vectors, viral vectors, mini-chromosomes, and analogs thereof. Examples of the viral vector include, but are not limited to, retroviruses, adenoviruses, and adeno-associated viruses.

Specifically, the vector may be plasmid DNA, phage DNA, etc., commercially developed plasmids (pUC18, pBAD, pIDTSAMRT-AMP, etc.), E. *coli*-derived plasmids (pYG601BR322, pBR325, pUC118, pUC119, etc.), *Bacillus subtilis-derived* plasmids (pUB110, pTP5, etc.), yeast-derived plasmids (YEp13, YEp24, YCp50, etc.), phage DNA (Charon4A, Charon21A, EMBL3, EMBL4, λgt10, λgt11, λZAP, etc.), animal viral vectors (retrovirus, adenovirus, vaccinia virus, etc.), insect viral vectors (baculovirus, etc.), and the like. Since the vector exhibits different protein expression levels and modifications depending on the host cell, it is preferable to select and use the most suitable host cell for the purpose.

In addition, the plasmid may contain a selectable marker such as an antibiotic resistance gene, and host cells maintaining the plasmid may be cultured under selective conditions.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vectors may contain a human cytomegalovirus (CMV) promoter for promoting continuous transcription of a target gene in mammalian cells, and a bovine growth hormone polyadenylation signal sequence for increasing the stability level of RNA after transcription.

### Transformed cell expressing fusion protein

In another aspect of the present invention, there is provided a transformed cell into which the expression vector comprising the polynucleotide encoding the fusion protein comprising an FcεRIα-ECD and a fragment of anti-IL-4R antibody is introduced.

As used herein, the term "transformed cell" refers to prokaryotic cells and eukaryotic cells into which a recombinant expression vector can be introduced. The transformed cell may be prepared by introducing a vector into a host cell and transforming it. In addition, the fusion protein of the present invention may be produced by expressing the polynucleotide contained in the vector.

The transformation may be performed by various methods. As long as it can produce the fusion protein of the present invention, it is not particularly limited thereto. Specifically, as the transformation method, CaCl₂ precipitation method, Hanahan method whose efficiency has been increased by using a reducing agent such as dimethyl sulfoxide (DMSO) in CaCl₂ precipitation method, electroporation, calcium phosphate precipitation method, protoplast fusion method, agitation method using silicon carbide fiber, agrobacteria mediated transformation method, transformation method using PEG, dextran sulfate, lipofectamine and dry/inhibition mediated transformation method, or the like may be used. In addition, by using the infection as a means, a target object can be delivered into a cell using virus particles. In addition, the vector can be introduced into the host cell by gene bombardment or the like.

In addition, as long as the host cell used for the production of the transformed cell can also produce the fusion protein of the present invention, it is not particularly limited thereto. Specifically, the host cells may include, but are not limited to, prokaryotic cells, eukaryotic cells, and cells of mammalian, plant, insect, fungal, or bacterial origin. As an example of the prokaryotic cells, E. coli may be used. In addition, as an example of the eukaryotic cells, yeast may be used. In addition, as the mammalian cells, CHO cells, F2N cells, COS cells, BHK cells, Bowes melanoma cells, HeLa cells, 911 cells, AT1080 cells, A549 cells, SP2/0 cells, human lymphoblastoid, NSO cells, HT-1080 cells, PERC.6 cells, HEK 293 cells, HEK293T cells, or the like may be used, but are not limited thereto, and any cells which are known to one of ordinary skill in the art to be usable as mammalian host cells may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those skilled in the art, glycosylation-associated genes possessed by host cells.

### Method of producing fusion protein

In another aspect of the present invention, there is provided a method of producing the fusion protein dimer comprising an FcεRIα-ECD and a fragment of anti-IL-4R antibody.

The method of producing the fusion protein may comprise: i) culturing the transformed cell; and ii) recovering the fusion protein dimer of the present invention comprising an FcεRIα-ECD and a fragment of anti-IL-4R antibody.

As used herein, the term "culture" refers to a method of growing microorganisms in an appropriately artificially controlled environmental condition.

The method of culturing the transformed cells may be carried out using a method well known in the art. Specifically, the culture is not particularly limited as long as it can express and produce the fusion protein of the present invention. Specifically, the culture may be carried out in a batch process, or carried out continuously in a fed batch or repeated fed batch process.

In addition, the recovering the fusion protein dimer from the culture may be carried out by a method known in the art. Specifically, the recovering method is not particularly limited as long as it can recover the produced fusion protein of the present invention. Preferably, the recovering method may be centrifugation, filtration, extraction, spraying, drying, evaporation, precipitation, crystallization, electrophoresis, fractional dissolution (for example, ammonium sulfate precipitation), chromatography (for example, ion exchange, affinity, hydrophobicity and size exclusion), and the like.

### Use of fusion protein

In another aspect of the present invention, there is provided a pharmaceutical composition for preventing or treating allergic diseases, comprising the fusion protein dimer comprising an FcεRIα-ECD and a fragment of anti-IL-4R antibody.

In the present specification, the term "allergic disease" means a pathological symptom caused by an allergic reaction mediated by mast cell activation, such as mast cell degranulation. Such allergic diseases include food allergy, atopic dermatitis, asthma, allergic rhinitis, allergic conjunctivitis, allergic dermatitis, allergic contact dermatitis, anaphylaxis, urticaria, pruritus, insect allergy, chronic idiopathic urticaria, chronic spontaneous urticaria, drug allergy, and the like. In particular, the allergic diseases may be IgE-mediated.

The term "prevention" refers to any action that inhibits the occurrence of or delays the onset of allergic diseases by administration of the pharmaceutical composition. The term "treatment" refers to any action that improves or beneficially changes the symptoms of allergic diseases by administration of the pharmaceutical composition.

In the pharmaceutical composition of the present invention for treating or preventing allergic diseases, the fusion protein dimer may be contained in any amount (effective amount) depending on the use, formulation, blending purpose, and the like, as long as it can exhibit an anti-allergic activity. A conventional effective amount may be determined within a range of 0.001 % by weight to 20.0 % by weight based on the total weight of the composition. Here, the "effective amount" refers to an amount of an active ingredient capable of inducing an anti-allergic effect. Such an effective amount may be experimentally determined within the scope of common knowledge of those skilled in the art.

Here, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffering agent, dispersing agent) may also be contained in the pharmaceutical composition.

Specifically, the pharmaceutical composition of the present invention contains, in addition to the fusion protein dimer, pharmaceutically acceptable carrier, and may be prepared into a parenteral formulation depending on a route of administration by a conventional method known in the art. Here, the term "pharmaceutically acceptable" means not having more toxicity than a subject to be applied (prescribed) can accommodate withoutinhibiting activity of the fusion protein dimer.

When the pharmaceutical composition of the present invention is prepared as an oral formulation, it may be prepared in the form of powders, granules, tablets, pills, dragees, capsules, liquids, gels, syrups, suspensions, wafers, and the like according to methods known in the art together with a suitable carrier. Here, examples of suitable pharmaceutically acceptable carriers may include sugars such as lactose, glucose, sucrose, dextrose, sorbitol, mannitol, and xylitol, starches such as corn starch, potato starch, and wheat starch, celluloses such as cellulose, methyl cellulose, ethyl cellulose, sodium carboxymethyl cellulose, and hydroxypropyl methyl cellulose, polyvinylpyrrolidone, water, methyl hydroxybenzoate, propyl hydroxybenzoate, magnesium stearate, mineral oil, malt, gelatin, talc, polyol, vegetable oil, and the like. In the case of formulation, if necessary, it may be formulated to include a diluent and/or excipient such as a filler, an extender, a binder, a wetting agent, a disintegrant, and a surfactant.

When the pharmaceutical composition of the present invention is prepared into a parenteral formulation, it may be formulated in the form of injections, transdermal drugs, nasal inhalers, and suppositories, together with suitable carriers, in accordance with methods known in the art. In a case of being formulated into injections, sterile water, ethanol, polyol such as glycerol or propylene glycol, or a mixture thereof may be used as a suitable carrier. For the carrier, isotonic solution, such as Ringer's solution, phosphate buffered saline (PBS) containing triethanolamine, or sterile water for injection, and 5% dextrose, and the like may be preferably used.

Formulation of pharmaceutical compositions is known in the art, and reference may specifically be made to Remington's Pharmaceutical Sciences (19th ed., 1995) and the like. This literature is considered part of the present specification.

On the other hand, the pharmaceutical composition of the present invention is administered in a pharmaceutically effective amount. As used herein, the term "administration" means introducing a predetermined substance to a subject by an appropriate method, and the composition may be administered through any general route as long as it can reach a target tissue. The route of administration may include oral administration, intraperitoneal administration, intravenous administration, intramuscular administration, subcutaneous administration, intradermal administration, topical administration, intranasal administration, and intrarectal administration, but is not limited thereto.

The term "pharmaceutically effective amount" refers to an amount that is sufficient to treat a disease with a reasonable benefit/risk ratio applicable to medical treatment and does not cause side effects. The level of the effective dose may be easily determined by those skilled in the art depending on factors including the sex, age, body weight, and health status of the patient, the type and severity of the disease, the activity of the drug, the sensitivity to the drug, administration method, administration time, the route of administration and excretion rate, treatment duration, the combined or concurrently used drugs and other factors well known in the medical field.

A preferable daily dosage of the pharmaceutical composition of the present invention may be in the range of 0.01 µg/kg to 10 g/kg per day, preferably 0.01 mg/kg to 1 g/kg per day, depending on the patient's condition, body weight, sex, age, disease severity, or route of administration. Administration may be carried out once or several times a day. Such a dosage should in no way be interpreted as limiting the scope of the present invention.

The term "subject" refers to a subject to which the composition of the present invention can be applied (prescribed), and may be a mammal, such as a human, a rat, a mouse, or a livestock. Preferably, it may be a human, but is not limited thereto. In addition to the fusion protein dimer, the anti-allergic composition of the present invention may further include any compound or natural extract known to have anti-allergic activity and have already proven safety for the enhancement and reinforcement of anti-allergic activity. Here, the fusion protein dimer and the compound or natural extract having anti-allergic activity may be administered simultaneously or sequentially.

In another aspect of the present invention, there is provided a food composition for improving or alleviating allergic symptoms, comprising the fusion protein dimer that comprises an FcεRIα-ECD and a fragment of anti-IL-4R antibody.

Here, the fusion protein dimer may be combined with an appropriate delivery means for efficient delivery into the intestine. In addition, the food composition of the present invention may be prepared in any form, and it may be prepared in the form of, for example, beverages such as tea, juice, carbonated beverage, and ionic beverage, processed dairy products such as milk and yogurt, health functional food formulations such as tablet, capsule, pill, granule, liquid, powder, flake, paste, syrup, gel, jelly, bar, and the like. In addition, the food composition of the present invention may fall within any product category in legal or functional classification as long as the food composition complies with the enforcement regulations at the time of being manufactured and distributed. For example, the food composition may be a health functional food according to the Health Functional Food Act, or it may be a confectionery, beans, tea, beverages, special purpose food, and the like according to each food type in the Food Standards Code of the Food Sanitation Act (Ministry of Food and Drug Safety Announcement, Food Standards and Specifications). In relation to other food additives that may be included in the food composition of the present invention, reference may be made to the Food Safety Code or the Food Additives Code according to the Food Sanitation Act.

In another aspect of the present invention, there is provided a use of the fusion protein dimer comprising an FcεRIα-ECD and a fragment of anti-IL-4R antibody for the manufacture of a medicament for the treatment or prevention of allergic diseases. Here, the FcεRIα-ECD, anti-IL-4R antibody, allergic disease, treatment and prevention are as described above.

In another aspect of the present invention, there is provided a use of the fusion protein dimer comprising an FcεRIα-ECD and a fragment of anti-IL-4R antibody for the treatment or prevention of allergic diseases. Here, the FcεRIα-ECD, anti-IL-4R antibody, allergic disease, treatment and prevention are as described above.

In another aspect of the present invention, there is provided a method for treating or preventing allergic diseases, comprising administering to a subject the fusion protein dimer comprising an FcεRIα-ECD and a fragment of anti-IL-4R antibody. Here, the FcεRIα-ECD, anti-IL-4R antibody, administration, allergic disease, treatment and prevention are as described above.

The subject may be a mammal, preferably a human. In addition, the subject may be a patient suffering from an allergic disease or may be a subject with a high probability of suffering from an allergic disease.

Route of administration, dosage, and frequency of administration of the fusion protein dimer comprising an FcεRIα-ECD and a fragment of anti-IL-4R antibody may vary depending on the patient's condition and the presence or absence of side effects, and thus it may be administered to a subject in various ways and amounts. The optimal administration method, dosage, and frequency of administration may be selected in an appropriate range by those skilled in the art. In addition, the fusion protein dimer comprising an FcεRIα-ECD and a fragment of anti-IL-4R antibody may be administered in combination with other drugs or physiologically active substances whose therapeutic effect is known with respect to a disease to be treated, or may be formulated in the form of combination preparations with other drugs.

### Mode for Carrying out the Invention

Hereinafter, the present invention will be described in more detail with reference to the following examples. These examples are with reference to illustrate the present invention, and it will be apparent to those skilled in the art that the scope of the present invention is not to be construed as being limited by these examples.

### Preparation Example 1. Preparation of FcεRIα ECD-Fc-anti-IL-4R scFv fusion protein: GI-305

In order to produce a fusion protein comprising an FcεRI α chain extracellular domain, an Fc domain and an antibody that specifically binds to the IL-4 receptor (IL-4R), a polynucleotide that comprises the nucleotide sequence (SEQ ID NO: 19) encoding a fusion protein comprising the FcεRI α chain extracellular domain (SEQ ID NO: 2), the linker (SEQ ID NO: 3), the IgG4 Fc domain (SEQ ID NO: 4), the linker (SEQ ID NO: 5) and the scFv of the antibody that specifically binds to the IL-4 receptor (SEQ ID NO: 6) in this order from the N-terminus was synthesized and loaded into the pcDNA3.4 vector (Genscript).

The vector was introduced into the CHO cells (ExpiCHO-S cells). Thereafter, the cells were cultured for 14 days in 37°C, 8% CO₂, serum-free ExpiCHO^{™} expression medium (Thermo Fisher Scientific). Thereafter, the culture solution was collected, and the fusion protein was purified using affinity chromatography (affinity purification column).

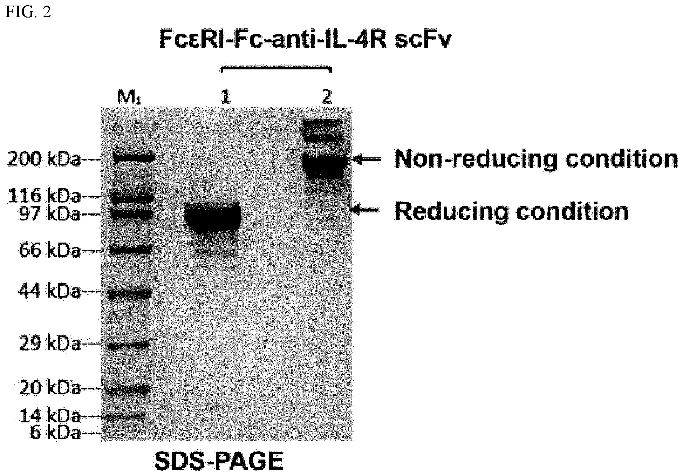

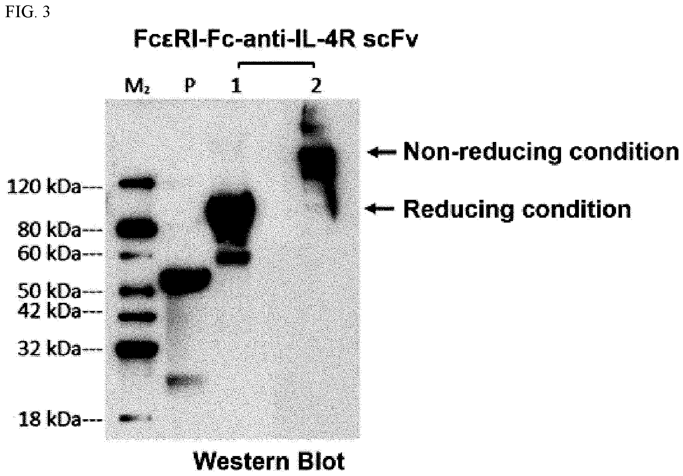

The molecular weight and purity of the purified fusion protein were confirmed by SDS-PAGE and Western blot. As a result of SDS-PAGE and Western blot analysis, it was confirmed that the protein was detected under non-reducing conditions and reducing conditions. From this, it was confirmed that the purified fusion protein forms a dimer (FIGs. 2 and 3). The fusion protein dimer was designated as "GI-305 (also referred to as FcεRIα ECD-Fc-anti-IL-4R scFv)".

### Preparation Example 2. Preparation of FcεRIα ECD-Fc fusion protein as control

### Preparation Example 2.1. Preparation of GI-301

In order to produce a fusion protein comprising an FcεRI α chain extracellular domain and an Fc domain as a control, a polynucleotide that comprises the nucleotide sequence (SEQ ID NO: 30) encoding a fusion protein comprising the FcεRI α chain extracellular domain (SEQ ID NO: 2), the linker (SEQ ID NO: 28) and the modified IgG4 Fc domain (SEQ ID NO: 29) in this order from the N-terminus was synthesized and loaded into the pcDNA3.4 vector (Genscript).

The vector was introduced into the CHO cells (ExpiCHO-S cells). Thereafter, the cells were cultured for 14 days in 37°C, 8% CO₂, serum-free ExpiCHO^{™} expression medium (Thermo Fisher Scientific). Thereafter, the culture solution was collected, and the fusion protein dimer was purified using affinity chromatography (affinity purification column).

### Preparation Example 2.2. Preparation of GI-305CN

In order to produce a fusion protein comprising an FcεRI α chain extracellular domain and an Fc domain, a polynucleotide that comprises the nucleotide sequence (SEQ ID NO: 31) encoding a fusion protein comprising the FcεRI α chain extracellular domain (SEQ ID NO: 2), the linker (SEQ ID NO: 3), and the IgG4 Fc domain (SEQ ID NO: 4) in this order from the N-terminus was synthesized and loaded into the pcDNA3.4 vector (Genscript).

The vector was introduced into the CHO cells (ExpiCHO-S cells). Thereafter, the cells were cultured for 14 days in 37°C, 8% CO₂, serum-free ExpiCHO^{™} expression medium (Thermo Fisher Scientific). Thereafter, the culture solution was collected, and the fusion protein dimer was purified using affinity chromatography (affinity purification column).

### Preparation Example 3. Preparation of Fc-anti-IL4Rα scFv as control: GI-305C1

In order to produce a fusion protein comprising an Fc domain and an antibody that specifically binds to the IL-4 receptor (IL-4R), a polynucleotide that comprises the nucleotide sequence (SEQ ID NO: 32) encoding a fusion protein comprising the IgG4 Fc domain (SEQ ID NO: 4), the linker (SEQ ID NO: 5) and the scFv (SEQ ID NO: 6) of the antibody that specifically binds to the IL-4 receptor in this order from the N-terminus was synthesized and loaded into the pcDNA3.4 vector (Genscript).

The vector was introduced into the CHO cells (ExpiCHO-S cells). Thereafter, the cells were cultured for 14 days in 37°C, 8% CO₂, serum-free ExpiCHO^{™} expression medium (Thermo Fisher Scientific). Thereafter, the culture solution was collected, and the fusion protein dimer was purified using affinity chromatography (affinity purification column).

### Experimental Example 1. Identification of binding ability of GI-305 fusion protein dimer to human IgE

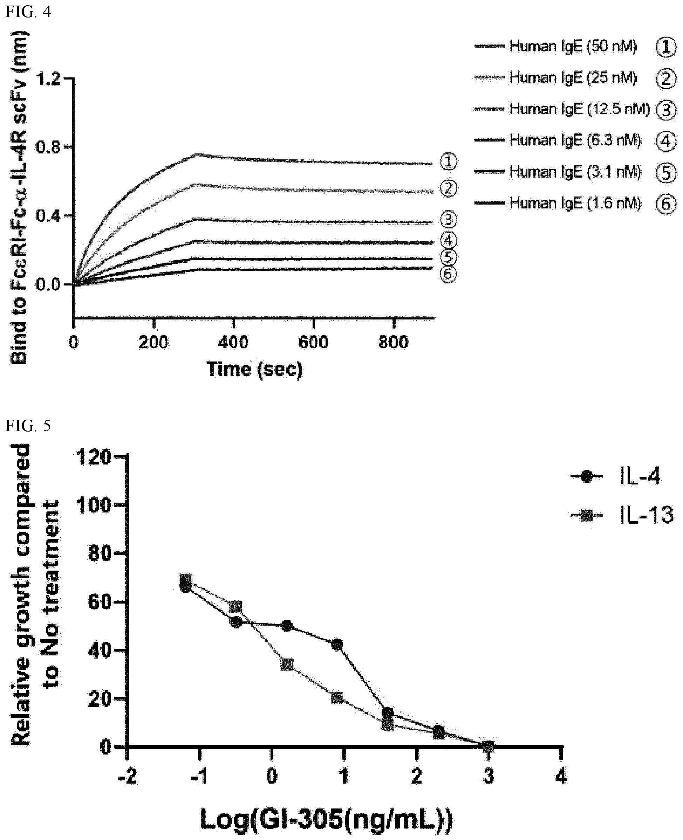

The binding ability to IgE was measured for the fusion protein GI-305 comprising an FcεRIα ECD and a fragment of anti-IL-4R antibody obtained through the method of Example 1 above. At this time, the binding ability to IgE was identified using Octet RED 384 (ForteBio). AHC (Anti-Human IgG Capture) biosensor was used by immersion in 1X kinetic buffer for 10 minutes. GI-305 was prepared at a concentration of 10 µg/mL and coated on the biosensor, and the binding ability was identified at each concentration by serially diluting IgE to 1.6 to 100 nM. As a result, the binding ability between the GI-305 fusion protein dimer and IgE was measured as shown in FIG. 4 and Table 2.

**[Table 2]**

| Kₐ (Association rate, 1/Ms) | K_{d} (Dissociation rate, 1/s) | K_{D} (K_{d}/Kₐ, M) |
|---|---|---|
| 2.01E+05 | 9.74E-05 | 0.5 nM |

### Experimental Example 2. Identification of IL-4 and IL-13 activity inhibitory effect of GI-305 fusion protein dimer

In order to identify the activity of the GI-305 fusion protein dimer as an anti-IL-4R antibody, an experiment was conducted using TF-1 cells, a cytokine-dependent cell line for cell growth. TF-1 cells are a cell line that responds to a number of cytokines including IL-4 and IL-13, and it was identified whether the GI-305 fusion protein dimer inhibited the cell proliferation mediated by IL-4 or IL-13 in TF-1 cells.

The TF-1 cells (ATCC, #CRL-2003) were cultured in the medium (RPMI-1640 + 10% FBS + 1% penicillin/streptomycin) containing human GM-CSF (4 ng/mL, R&D Systems). Prior to analysis, the TF-1 cells were precipitated by centrifugation at 1,500 rpm for 5 minutes, and the medium was removed, and then the cells were resuspended in an assay medium without GM-CSF. The resuspended cells were aliquoted into each well of a 96-well plate at a density of 5×10⁴ cells/well. The GI-305 fusion protein dimer at various concentrations was mixed with IL-4 (100 ng/mL) and IL-13 (100 ng/mL), respectively, and added to the wells. The assay plates were incubated at 5% CO₂, 37°C for 24 hours. Thereafter, 10 µL of WST-1 (Roche) was added to each well and incubated for 4 hours.

Thereafter, absorbance at a wavelength of 450 nm was recorded using a plate reader, and data was analyzed using GraphPad Prism software. As a result, it was shown that GI-305 inhibited the TF-1 cell growth induced by IL-4 or IL-13 (FIG. 5).

### Experimental Example 3. Identification of serum IgE level-reducing effect by GI-305 fusion protein dimer

IL-4 and IL-13 promote B cell proliferation, co-stimulate CD40/CD40L, and induce class switching in IgG4 and IgE. The effect of the GI-305 fusion protein dimer on the release of IgE from B cells induced by IL-4 was evaluated. Peripheral blood mononuclear cells (PBMCs) were purchased from Cellular Technology Limited, and an experiment was conducted. PBMCs were cultured in an assay medium (RPMI-1640 + 10% FBS + 1% penicillin/streptomycin).

Prior to analysis, PBMCs stored in the LN₂ tank were precipitated by centrifugation at 1,500 rpm for 5 minutes, and the medium was removed by suction, and then the cells were resuspended in an assay medium. The cells were aliquoted into each well of a 96-well plate in an assay medium at a density of 2×10⁵ cells/well. The commercially available anti-IgE antibody omalizumab (product name: Xolair) and the anti-IL-4Rα antibody dupilumab (product name: Dupixent) as a control, GI-301 (FcεRIα ECD-modified Fc), GI-305CN (FcεRIα ECD-IgG4 Fc), and GI-305C1 (IgG4 Fc-IL-4RαscFv), which were prepared in the above preparation examples, and the test substance GI-305 were each mixed with IL-4 (30 ng/mL) and the anti-human CD40 antibody (1 µg/mL) and added to wells of the plate. The control and the test substance were prepared and used at a concentration of 0.008 nM or 0.04 nM. The assay plates were cultured at 5% CO₂, 37°C for 12 days. Thereafter, it was precipitated by centrifugation at 1,500 rpm, and the level of IgE present in the supernatant was measured using an R-PLEX assay kit (MSD).

Specifically, 25 µL of biotinylated capture antibody was aliquoted into each well of MSD GOLD 96-well Small Spot Streptavidin Plates. After incubation for 1 hour at room temperature, each well was washed 5 times with 150 µL of PBST (PBS containing 0.05% Tween 20). Thereafter, 25 µL of the sample was put into each well, and then incubated at room temperature for 1 hour. Then, it was washed 5 times with 150 µL of PBST per well. After washing, 150 µL of sulfo-tag detection antibody was aliquoted into each well. After incubation for 1 hour at room temperature, it was washed 5 times with 150 µL of PBST per well. Thereafter, 150 µL of MSD GOLD read buffer was aliquoted into each well and measured with MESO QuickPlex SQ 120. Data was analyzed using GraphPad Prism software.

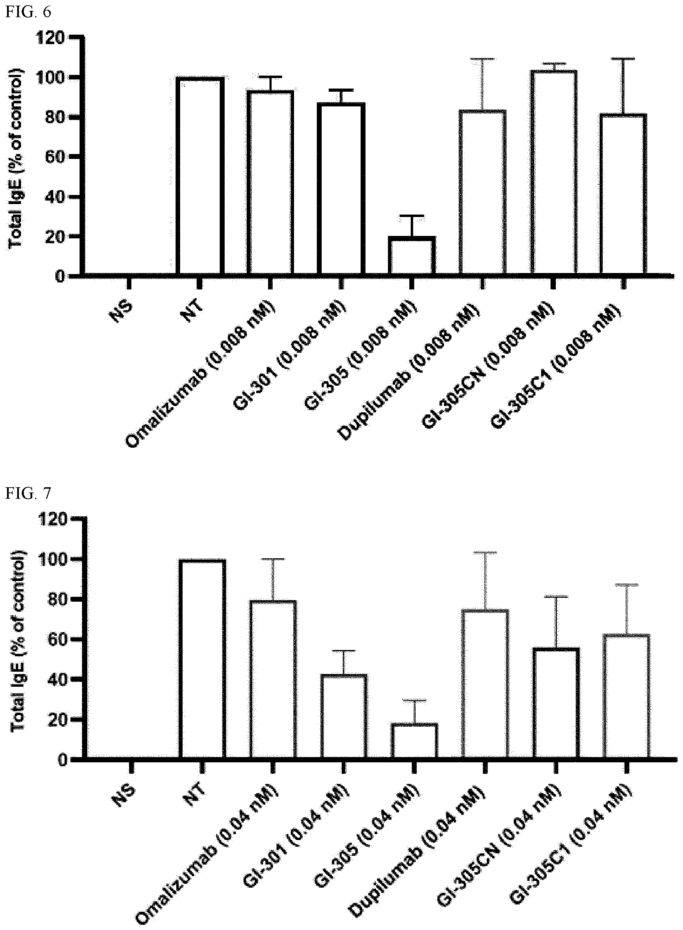

As a result, it was found that the GI-305 fusion protein dimer exhibited a significantly excellent effect in inhibiting IgE production through inhibition of B cell activity compared to the anti-IgE antibody omalizumab, the anti-IL-4Rα antibody dupilumab, GI-301 (FcεRIα ECD-modified Fc), GI-305CN (FcεRIα ECD-IgG4 Fc) and GI-305C1 (IgG4 Fc-IL-4RαscFv) (FIGs. 6 and 7).

## Claims

1. A fusion protein comprising an IgE Fc receptor alpha subunit extracellular domain (FcεRIα-ECD) and a fragment of anti-IL-4R antibody.

2. The fusion protein according to claim 1, wherein the fusion protein comprises an Fc region of an immunoglobulin.

3. The fusion protein according to claim 1, wherein the IgE Fc receptor alpha subunit extracellular domain consists of the amino acid sequence of SEQ ID NO: 2 or a fragment thereof.

4. The fusion protein according to claim 1, wherein the fragment of anti-IL-4R antibody comprises
a heavy chain variable region comprising HCDR1 of SEQ ID NO: 20, HCDR2 of SEQ ID NO: 21 and HCDR3 of SEQ ID NO: 22; and
a light chain variable region comprising LCDR1 of SEQ ID NO: 23, LCDR2 of SEQ ID NO: 24 and SEQ ID NO: 25.

5. The fusion protein according to claim 1, wherein the fragment of anti-IL-4R antibody comprises a heavy chain variable region of SEQ ID NO: 7 and a light chain variable region of SEQ ID NO: 8.

6. The fusion protein according to claim 5, wherein the heavy chain variable region of SEQ ID NO: 7 and the light chain variable region of SEQ ID NO: 8 are linked by a peptide linker.

7. The fusion protein according to claim 1, wherein the fusion protein consists of the following structural formula (I) or (II):
N'-X-linker (1)-Fc region fragment or variant thereof-linker (2)-Y-C' (I)
N'-Y-linker (1)-Fc region fragment or variant thereof-linker (2)-X-C' (II)
in the structural formulas (I) and (II),
N' is the N-terminus of the fusion protein,
C' is the C-terminus of the fusion protein,
X is FcεRIα-ECD or a fragment thereof,
Y is a fragment of anti-IL-4R antibody, and
the linker (1) and linker (2) are peptide linkers.

8. The fusion protein according to claim 7, wherein the Fc region of the fusion protein is derived from human IgG4.

9. A fusion protein dimer in which two fusion proteins according to any one of claims 1 to 8 are combined.

10. A polynucleotide encoding the fusion protein according to any one of claims 1 to 8.

11. An expression vector comprising the polynucleotide of claim 10.

12. A transformed cell into which the vector of claim 11 is introduced.

13. A method of producing a fusion protein dimer, comprising:
i) culturing the transformed cell of claim 12; and
ii) recovering a fusion protein dimer.

14. A pharmaceutical composition for preventing or treating allergic diseases, comprising the fusion protein dimer of claim 9.

15. The pharmaceutical composition according to claim 14, wherein the allergic disease is one selected from the group consisting of food allergy, atopic dermatitis, asthma, allergic rhinitis, allergic conjunctivitis, allergic dermatitis, chronic idiopathic urticaria, and allergic contact dermatitis.

16. A food composition for improving or alleviating allergic symptoms, comprising the fusion protein dimer of claim 9.

17. A use of the fusion protein dimer of claim 9 for the manufacture of a medicament for the treatment or prevention of allergic diseases.

18. A use of the fusion protein dimer of claim 9 for the treatment or prevention of allergic diseases.

19. A method for treating or preventing allergic diseases, comprising administering to a subject the fusion protein dimer of claim 9.
